# EUROPEAN PATENT APPLICATION

(11) **EP 4 752 111 A1**
(43) Date of publication of application: **03.06.2026**
(21) Application number: 25217863.7
(22) Date of filing: 24.11.2025
(51) Int. Cl.: C02F 1/32, A61L 9/20, B01D 53/00

(54) **AN IN-LINE UV-LED FLUID DISINFECTION UNIT AND A METHOD**

(30) Priority: 28.11.2024 SE 2451208
(71) Applicant: Watersprint AB, 223 81 Lund (SE)
(72) Inventor: Uvnäs, Krister, 24735 Södra Sandby (SE); Sandberg, Torbjörn, 21611 Limhamn (SE)
(74) Representative: Hansson Thyresson AB

(57) **Abstract**

An in-line UV-LED fluid disinfection unit (10) is provided, comprising a housing (11), a hollow structure (16) comprising a lateral surface, the hollow structure (16) being arranged in a cavity (14) of the housing (11) such that said cavity (14) is divided into an inner chamber (17) and an outer chamber (18). The unit (10) further comprises a UV-LED unit (15), and a sleeve (21) arranged in the outer chamber (18). The sleeve (21) encloses a portion of the lateral surface of the hollow structure (16). The sleeve (21) is provided with separation means (22) dividing the outer chamber (18) into a non-disinfected fluid chamber (23) and a disinfected fluid chamber (24). The separation means (22) comprises a sealing means (32) arranged such that a first portion (32a) of the sealing means (32) is arranged adjacent a first side portion of the fluid inlet opening (12), and a second portion (32b) of the sealing means (32) is arranged adjacent a second side portion of the fluid outlet opening (13), being opposite said first side portion.

## Description

### TECHNICAL FIELD

The present invention relates to a UV-LED fluid disinfection unit, and a method for disinfecting a fluid with such unit.

### BACKGROUND

Decontamination or disinfection by means of ultraviolet (UV) light is a well-established and reliable technique for use in systems for purifying fluids, in particular systems suitable for providing potable water. These systems are efficient and perform satisfactory without the use of any chemicals.

The UV disinfection unit may be used on its own, or in combination with filters. The UV disinfection unit may be retrofitted into an existing system, e.g. by replacing an old filter unit. Piping installations are commonly designed to accommodate a certain, traditional design of filter units, the units having inlet and outlet in one, common plane, and a filter compartment extending below the plane of the inlet/outlet, in the installed position. This is beneficial for a filter unit since such unit often has a drain valve arranged in its bottom portion. Since installers and pipework designers are used to and familiar with the design and installation of such filter units, it would be beneficial to provide a UV fluid disinfection unit which can be installed in the same manner, preferably without having to alter the piping design.

From the above it is understood that there is room for improvements and the invention aims to solve or at least mitigate the above and other problems.

### SUMMARY

The invention is defined by the appended independent claims. Additional features and advantages of the concepts disclosed herein are set forth in the description which follows, and in part will be obvious from the description, or may be learned by practice of the described technologies. The features and advantages of the concepts may be realized and obtained by means of the instruments and combinations particularly pointed out in the appended claims. These and other features of the described technologies will become more fully apparent from the following description and appended claims, or may be learned by the practice of the disclosed concepts as set forth herein.

In a first aspect a UV-LED fluid disinfection unit is provided, comprising a housing enclosing a cavity, and comprising an inlet connection and an outlet connection, a fluid inlet opening and a fluid outlet opening. The fluid inlet opening fluidly connects the inlet connection and the cavity, and the fluid outlet opening fluidly connects the cavity and the outlet connection. The inlet connection and the outlet connection are arranged in-line with each other.

The UV-LED fluid disinfection unit further comprises a hollow structure comprising a lateral surface, a first end portion and a second end portion. The hollow structure is arranged in the cavity such that said cavity is divided into an inner chamber and an outer chamber.

The UV-LED fluid disinfection unit further comprises a UV-LED unit arranged in connection with one end portion of the hollow structure and a sleeve arranged in the outer chamber. The sleeve encloses a portion of the lateral surface of the hollow structure, and/or the UV-LED unit. The sleeve is provided with separation means dividing the outer chamber in a non-disinfected fluid chamber and a disinfected fluid chamber and comprises a sealing means. The sleeve is arranged such that a first portion of the sealing means is arranged adjacent a first side portion of the fluid inlet opening, and a second portion of the sealing means is arranged adjacent a second side portion of the fluid outlet opening, being opposite said first side portion.

This UV-LED fluid disinfection unit is beneficial in that it provides for an in-line installation. The means that the inlet and outlet connections are arranged approximately on a straight line. This facilitates installation of the unit.

In one embodiment, the inlet connection and the outlet connection are arranged on a common centre axis. The definition of common centre axis includes a certain installation tolerance, such as e.g. +/- 5 mm off centre. The definition of common centre axis includes also a certain angular tolerance such as +/- 2°.

In one embodiment, the sleeve is arranged such that a first portion of the sealing means is arranged above the fluid inlet opening, and a second portion of the sealing means is arranged below the fluid outlet opening.

In one embodiment, the first side portion of the fluid inlet opening is an upper side portion, and the second side portion of the fluid outlet opening is a lower side portion.

In one embodiment, the fluid inlet opening is connected to the non-disinfected fluid chamber. In one embodiment, the fluid outlet opening is connected to the disinfected fluid chamber. The non-disinfected fluid chamber and the disinfected fluid chamber are not in direct fluid communication with each other. Fluid cannot pass directly between the non-disinfected fluid chamber and the disinfected fluid chamber. Fluid entering the UV-LED fluid disinfection unit is led to the non-disinfected fluid chamber portion. The fluid exits the UV-LED fluid disinfection unit from the disinfected fluid chamber.

In one embodiment, an interior of the hollow structure forms the inner chamber, and/or the outer chamber comprises a space formed radially outside the hollow structure and radially inside the housing. In one embodiment, the outer chamber is delimited by an outer wall of the hollow structure and an inner wall of the housing. The inner and outer chambers are arranged one within the other. This provides for a compact design of the UV-LED fluid disinfection unit.

In one embodiment, the second portion of the sealing means is circumferentially opposite the first portion of the sealing means. This provides for a symmetrical design of the unit.

In one embodiment, the UV-LED unit comprises a casing and a lid. The lid may comprise at least one UV transparent portion. In one embodiment, the casing and the lid form an enclosed space housing one or more of: one or more UV-LED(s), and one or more PCB(s). This is a favourable way of designing the UV-LED unit in that it is easy to assemble and manufacture. Possibly, the lid is removable from the casing such that the components housed therein are exchangeable.

In one embodiment, the PCB is provided with a cooling medium, optionally a cooling paste, optionally on a surface of the PCB facing the casing. The cooling medium is beneficial in that it provides cooling to the PCB and/or the LEDs arranged thereon.

In one embodiment, the UV-LED(s) are configured to transmit UV light into the inner chamber. The inner chamber may be a fluid disinfection chamber.

In one embodiment, the separation means comprises a flange with a groove housing the sealing means. This provides an efficient separation means. In one embodiment, the separation means is provided obliquely around the sleeve. This provides for the non-disinfected fluid chamber and the disinfected fluid chamber, being separated by the separation means, are asymmetrical. This in turns provides for an inline installation of the unit, since the inlet and outlet connections, leading to/from the non-disinfected fluid chamber and the disinfected fluid chamber respectively, may be arranged on a common axis.

In one embodiment, the sealing means comprises on or more of: a ring-shaped sealing, optionally an O-ring or a sealing with any other suitable cross section geometry; an injection moulded sealing, a sealing integrally formed with the sleeve. This sealing means provides for efficient sealing between the non-disinfected fluid chamber and the disinfected fluid chamber, such that fluid cannot pass directly between these chambers.

In a second aspect, a method for disinfecting a fluid is provided. The method comprises: providing a UV-LED fluid disinfection unit; providing a flow of fluid through the inlet opening into the non-disinfected fluid chamber; directing the fluid, by means of the separation means, towards one end portion of the hollow structure, where the fluid enters the inner chamber, and flows toward the opposite end portion of the hollow structure; disinfecting the fluid in the inner chamber by means of UV radiation emitted from the UV-LED unit; directing the fluid into the disinfected fluid chamber; directing the fluid, by means of the separation means, towards the outlet opening.

In one embodiment, the fluid contacts an exterior of the UV-LED unit, whereby the fluid cools the UV-LED unit. The cooling effect is enhanced if the UV-LED unit comprises a cooling medium such as a cooling paste. These are efficient ways of providing cooling to the UV-LED unit, and especially the PCB and/or the thereon provided LEDs.

In one embodiment, the hollow structure is a reflector unit. This is beneficial for enhanced dispersion of the UV light inside the disinfection chamber.

In one embodiment, the casing comprises stainless steel and/or the at least one UV transparent portion of the lid comprises quartz glass. These are beneficial materials to use as they are UV transparent and fluid, especially water resistant.

In one embodiment, the UV-LED fluid disinfection unit further comprises a flow distributor arranged in an opposite end portion of the hollow structure compared to the UV-LED unit. The flow distributor is beneficial in that it provides an advantageous distribution of the flow of fluid within the inner chamber, i.e. within the disinfection chamber. Thus, an as large portion as possible of the fluid to be disinfected is exposed to the UV light.

In one embodiment, the sleeve peripherally encloses the hollow structure.

### BRIEF DESCRIPTION OF THE DRAWINGS

In order to best describe the manner in which the above-described embodiments are implemented, as well as define other advantages and features of the disclosure, a more particular description is provided below and is illustrated in the appended drawings. Understanding that these drawings depict only exemplary embodiments of the invention and are not therefore to be considered to be limiting in scope, the examples will be described and explained with additional specificity and detail through the use of the accompanying drawings in which:
- Fig. 1: is a perspective view of a UV-LED fluid disinfection unit according to one embodiment;
- Fig. 2: is a front view of the UV-LED fluid disinfection unit in Fig. 1;
- Fig. 3: is a side view of the UV-LED fluid disinfection unit in Fig. 1;
- Fig. 4: is a section view taken along line A-A of the UV-LED fluid disinfection unit in Fig. 3;
- Fig. 5: is a perspective view of a sleeve according to one embodiment;
- Fig. 6: is a front view of the sleeve in Fig. 5;
- Fig. 7: is a side view of the sleeve in Fig. 5;
- Fig. 8: is a top view of the sleeve in Fig. 5;
- Fig. 9: is a partial perspective view of the UV-LED fluid disinfection unit in Fig. 4;
- Fig. 10: is a partial perspective view of the UV-LED fluid disinfection unit in Fig. 4; and
- Fig. 11: is a partial section view a UV-LED fluid disinfection unit according to one embodiment.

Further, in the figures like reference characters designate like or corresponding parts throughout the several figures.

### DETAILED DESCRIPTION

Various embodiments of the disclosed methods and arrangements are discussed in detail below. While specific implementations are discussed, it should be understood that this is done for illustration purposes only. A person skilled in the relevant art will recognize that other components, configurations, and steps may be used without parting from the spirit and scope of the disclosure.

In the description and claims the word "comprise" and variations of the word, such as "comprising" and "comprises", does not exclude other elements or steps.

Hereinafter, certain embodiments will be described more fully with reference to the accompanying drawings. It will be apparent to those skilled in the art that various modifications and variations can be made without departing from the inventive concept. Other embodiments will be apparent to those skilled in the art from consideration of the specification and practice disclosed herein. The embodiments herein are provided by way of example so that this disclosure will be thorough and complete, and will fully convey the scope of the inventive concept, and that the claims be construed as encompassing all equivalents of the present inventive concept which are apparent to those skilled in the art to which the inventive concept pertains. If nothing else is stated, different embodiments may be combined with each other.

Many kinds of UV disinfection units for disinfection of a fluid such as water, e.g. drinking water or tap water, are available. Typically, a UV-lamp disinfection unit is generally cylindric in shape and has an inlet in a top/bottom area thereof, and an outlet in a lateral surface, distal from the top/bottom area in which the inlet is provided. Other products have both the inlet and outlet provided in a common side of the lateral surface. The configuration of these types of units requires a pipe installation comprising a number of bends. This is cumbersome for installers, does not provide a "clean" finish of the piping installation and requires space.

For installation purposes, an in-line UV disinfection unit is beneficial. One known way of providing an inline product is to design a unit resembling a cylinder with a longitudinal axis corresponding to the axis of extension of the pipe in which the unit is installed. I.e., the fluid maintains the same direction inside the unit as it had when it entered the unit. However, this configuration is bulky and takes up much space. It may be hard to fit such unit into an existing pipe installation.

The present device has a main extension in a direction perpendicular to the direction of the pipe leading into and out of the unit. The fluid is thus redirected inside the unit. This enables a compact design which is easier to install, and especially to retrofit into an existing pipe system, for example an installation not previously comprising a UV-disinfection unit. This design resembles a traditional filter unit, which the installers are familiar with and know how to handle and install in an efficient manner. This kind of unit does not require any additional piping bends and can be installed next to a filter unit. This provides a clean installation with a coherent appearance.

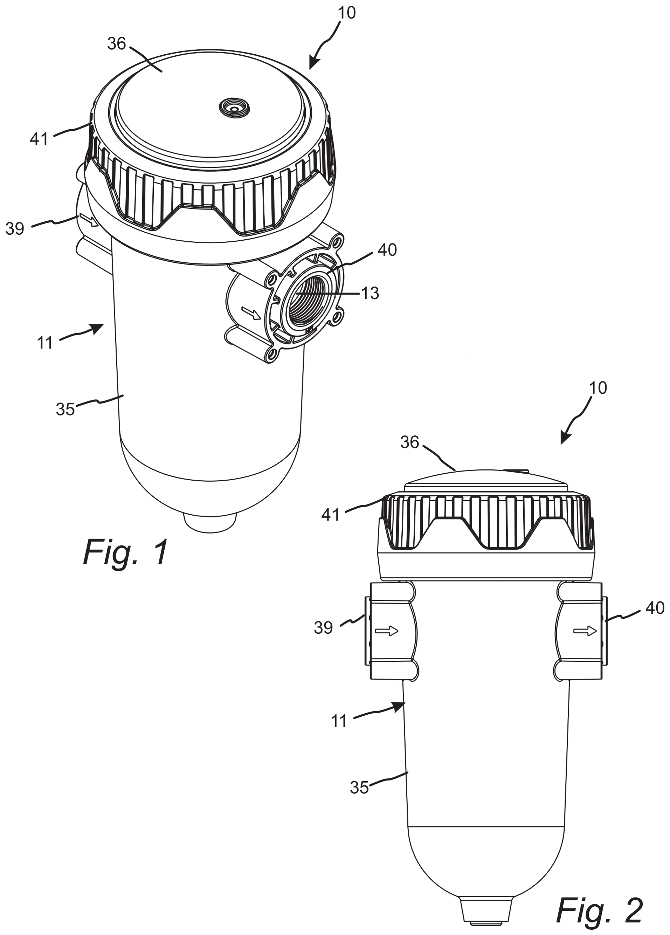

In Figs 1-3, a UV-LED fluid disinfection unit 10 is show. The UV-LED fluid disinfection unit 10 is configured to be installed in a pipe system for a fluid. It is configured to disinfect the fluid transported via the pipe system by means of UV radiation. The UV-LED fluid disinfection unit 10 is configured to have an in-line installation configuration. This means that an inlet connection 39 and an outlet connection 40 of the unit 10 are provided in-line with each other, i.e. being provided on a common axis 25.

The unit 10 comprises a housing 11 which comprises a container portion 35 and a cover portion 36. The container portion 35 and the cover portion 36 are releasably connected to each other. This is beneficial for maintenance purposes, enabling exchange of components housed within the housing 11. The container portion 35 and the cover portion 36 are attach to each other by means of a nut 41.

The unit 10 is provided with an inlet connection 39 having a fluid inlet opening 12, and an outlet connection 40 having a fluid outlet opening 13. The inlet connection 39 and the outlet connection 40 are configured to be connected to fluid pipes, transporting the fluid to be disinfected.

The inlet connection 39 and the outlet connection 40 are arranged in level with one another, i.e. a distance between a top of the unit 10 and the inlet connection 39 equals a distance from the top of the unit 10 and the outlet connection 40. The inlet connection 39 and the outlet connection 40 are separated by approximately 180° around the periphery of the housing 11.

Fluid to be disinfected is to be entered into the unit 10 via the fluid inlet 12, and disinfected fluid is to be discharged via the outlet 13. The fluid inlet opening 12 and the fluid outlet opening 13 are, in the shown embodiment, arranged on the common axis 25. The openings 12, 13 are also shown as extending straight along the axis 25. In other embodiments however, the fluid inlet opening 12 and the fluid outlet opening 13 may not share a common centre axis. They may also have an extension which is oblique in relation to the axis 25.

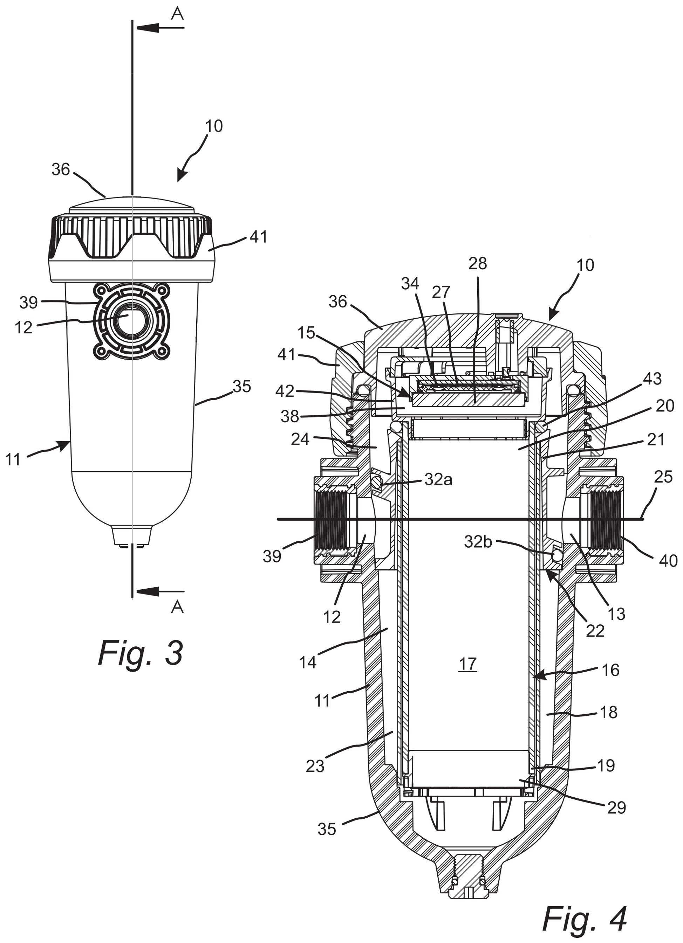

Referring to Fig. 4, a section view taken along line A-A in Fig. 3 is shown. The container portion 35 forms together with the cover portion 36 an enclosed space 14 or cavity. In Fig. 4, the fluid inlet 12 and the fluid outlet 13 are arranged in the container portion 35, but may in other embodiments be arranged in the cover portion 36.

Within the cavity 14, a hollow structure 16 is arranged. The hollow structure 16 is elongate and comprises a lateral surface. The hollow structure 16 comprises a first end portion 19 and a second end portion 20, located in opposite ends of the lateral surface. The first end portion 19 and the second end portion 20 are open end portions. The end portions 19, 20 could alternatively be semi-closed end portions, or be provided with one or more openings. The fluid is configured to flow in and out of the hollow structure 16 via said end portions.

In the shown embodiment, the hollow structure 16 comprises a cylinder structure. In other embodiments, the hollow structure 16 may have any other suitable shape such as rectangular, oval etc. It may have any suitable cross section, such as polygonal, square, oval, rectangular, irregular, regular etc. The cross section may be constant along the lateral surface of the structure 16, or it may vary along the lateral surface of the structure 16.

The hollow structure has a diameter being less than the inner diameter of the housing 11. Thus, an inner chamber 17, below also referred to as fluid disinfection chamber is formed within the hollow structure 16, and an outer chamber 18 is formed between an inner wall of the housing 11 and the lateral surface of the hollow structure 16. The outer chamber 18 is ring shaped, seen from a top/bottom section view of the disinfection unit 10. The outer chamber 18 extends circumferentially around the inner chamber 17.

In the first end portion 19 of the hollow structure 16, a flow distributor 29 is arranged. The flow distributor 29 comprises e.g. one or more strainers or sieves, one or several more or less rigid mesh net(s), a throttle means, or any other suitable flow distributing structure. The flow distributor 29 provides an advantageous distribution of the flow of fluid within the inner chamber 17. The target is to illuminate every fraction, or as close to every fraction as possible, of the fluid by the UV-light. The more distributed fluid flow promotes an enhanced disinfection of the fluid.

In the embodiment depicted in Fig. 4, a reception means 42 is arranged in the second end portion 20 of the hollow structure 16. The reception means 42 is configured to receive a UV-LED unit 15. The UV-LED unit 15 is attached to the cover portion 36. When the cover portion 36 is attached to the container portion 35, the UV-LED unit 15is received, together with the cover portion 36, into the reception means 42. Alternatively, the fluid disinfection unit does not comprise a reception means. In this case, the arrangement of the UV-LED unit is accomplished in any other suitable manner.

Together, the hollow structure 16, including the distribution means, and the reception means 42 are exchangeable, configured to be replaceable as a spare part of the fluid disinfection unit 10.

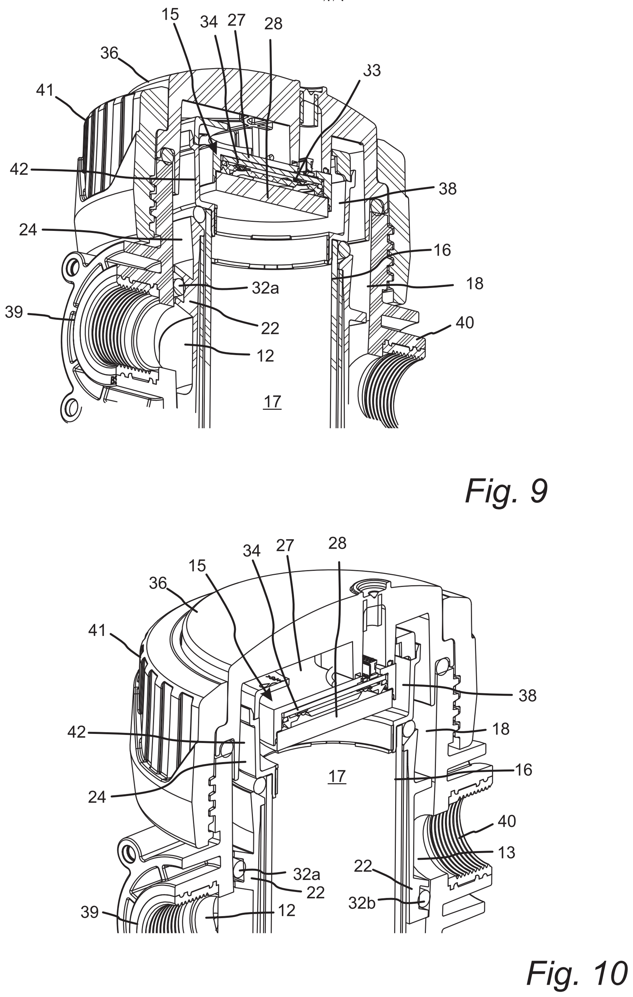

As best seen in Figs 9-10, the UV-LED unit 15 comprises a casing or housing 27 closed by a lid or cover 28. The casing 27 is e.g. made of stainless steel. The cover 28 is e.g. made of quartz glass. Together, the housing 27 and the cover 28 form an enclosed space, within which one or more UV-LEDs 33 and a PCB 34 are arranged. In one embodiment, the UV-LEDs are UVC-LEDs.

The PCB 34 may be arranged adjacent the casing 27. A cooling paste may be applied on the surface of the PCB 34 facing the casing 27. The UV-LEDs 33 are arranged on the opposite surface of the PCB 34.

The UV-LED unit 15 is arranged within the reception means 42 such that a fluid passage 38 is formed between an inner wall of the reception means 42and the exterior of the UV-LED unit 15. This fluid passage 38 connects the inner chamber 17 and the outer chamber 18.

Referring again to Fig. 4, the unit 10 further comprises a sleeve 21 arranged in the outer chamber 18. The sleeve 21 is arranged radially between the housing 11 and the hollow structure 16. In one embodiment, the sleeve 21 is arranged around the periphery of the hollow structure 16. In one embodiment, the sleeve 21 is arranged around the periphery of the reception means 42. In one embodiment, the sleeve 21 is arranged partly around the periphery of the reception means 42and partly around the hollow structure 16. The sleeve 21 encloses the circumference of the hollow structure 16 and/or the reception means 42.

The sleeve 21 is configured to fit snugly against the periphery of the hollow structure 16 (and/or reception means 42). Additionally, a sealing 43 such as an O-ring, is arranged between the sleeve 21 and the hollow structure 16 (and/or reception means 42). Fluid cannot pass between the sleeve 21 and the hollow structure 16, and cannot thus pass between a lower portion of the outer chamber 18 (referred to below as non-disinfected fluid chamber 23) and an upper portion of the outer chamber 18 (referred to below as disinfected fluid chamber 24) without passing the disinfection chamber 17.

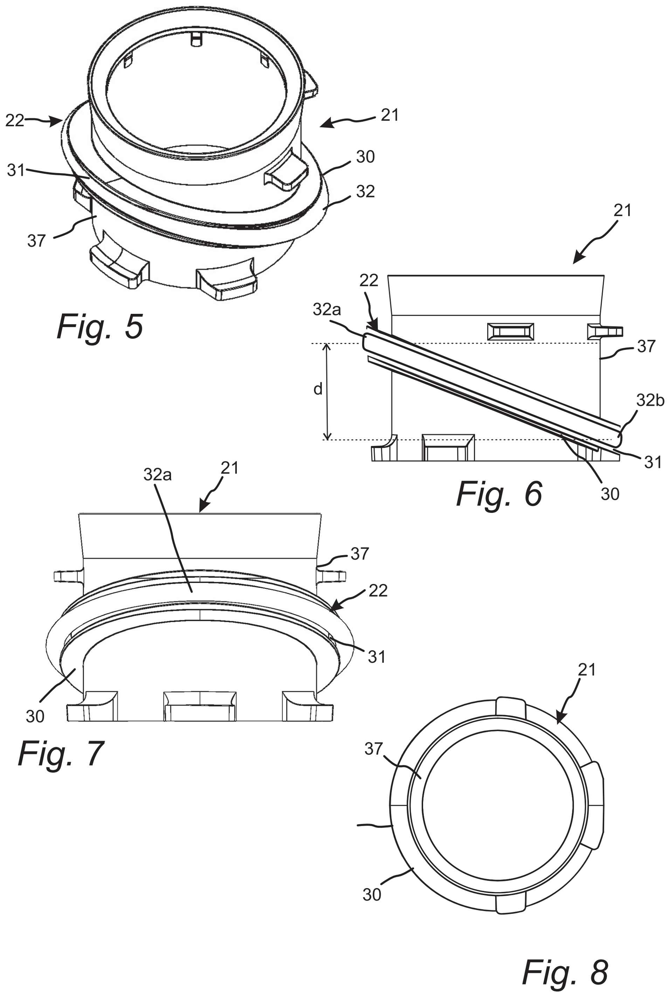

Referring also to Figs 5-8, the sleeve 21 comprises a cylinder-shaped body 37. In other embodiments, where the hollow structure 16 may have a differently shaped periphery or circumference. In that case, the sleeve 21 may have a corresponding periphery or circumference, or it may have a differently shaped periphery, or circumference. The sealing between the hollow structure 16 and the sleeve 21 is accomplished by one or more sealings 43.

The sleeve 21 comprises a flange 30 arranged obliquely around the outer periphery of the sleeve body 37. The flange 30 comprises a groove 31, configured to house a sealing means 32. The sealing means 32 may for example be a ring sealing, such as an O-ring, or a ring with any other suitable cross section. When arranged in the outer chamber 18, the sealing means 32 abut the inner wall of the housing 11 such that no fluid can pass. The sealing means 32 comprises a first portion 32a and a thereto opposite second portion 32b.

Seen in a front view, as in Fig. 6, a vertical distance d between the first portion 32a of the sealing means 32 and the second portion 32b of the sealing means 32 is at least equal to a diameter of the fluid inlet opening 12 and/or fluid outlet opening 13.
The flange 30 and the sealing means 32 thus form a separation means 22. The separation means 22 separates the outer chamber 18 into a first and a second portion, hereafter referred to as a non-disinfected fluid chamber 23 and a disinfected fluid chamber 24, respectively. The non-disinfected fluid chamber 23 is located in the area of/adjacent/in the vicinity of the first end portion 19 of the hollow structure 16. The non-disinfected fluid chamber 23 thus comprises the first end portion 19 of the hollow structure 16.

The disinfected fluid chamber 24 is located in the area of/adjacent/in the vicinity of the second end portion 20 of the hollow structure 16.

The sleeve 21 is arranged in the area of the inlet/outlet openings 12, 13 of the disinfection unit 10. The sleeve 21 is arranged such that a first portion 32a of the sealing means 32 is arranged on a first side of the inlet opening 12, and a second portion 32b of the sealing means 32 is arranged on a thereto opposite side of the outlet opening 13. With reference to the direction of Fig. 4, this corresponds to the first portion 32a of the sealing means 32 being arranged above the inlet opening 12, and the second portion 32b of the sealing means 22 being arranged below the outlet opening 13. In the shown embodiment, the first portion 32a of the sealing means 22 is arranged on a first side of the axis 25, and the second portion 32b of the sealing means 22 is arranged on an opposite side of the axis 25.

The inlet opening 12 is thus in fluid communication with the bottom portion, with reference to the direction of Fig. 4, of the outer chamber 18, i.e. the non-disinfected fluid chamber 23. The outlet opening 13 is in fluid communication with the top portion of the outer chamber 18, with reference to the direction of Fig. 4, i.e. the disinfected fluid chamber 24. Thus, a fluid passage is formed from the inlet opening 12, into the non-disinfected fluid chamber 23, into the disinfection fluid chamber 17, via the fluid passage 38 around the UV-LED unit 15, into the disinfected fluid chamber 24 and to the outlet opening 13.

In other embodiments, not shown, the distance d could be larger or smaller than the diameter of the inlet/outlet openings 12, 13. As an alternative, the inlet opening 12 is arranged above the separation means 22 and the outlet opening 13 is arranged below the separation means 22. According to this alternative, the disinfected and non-disinfected fluid chambers have changed places, compared to the embodiment described above.

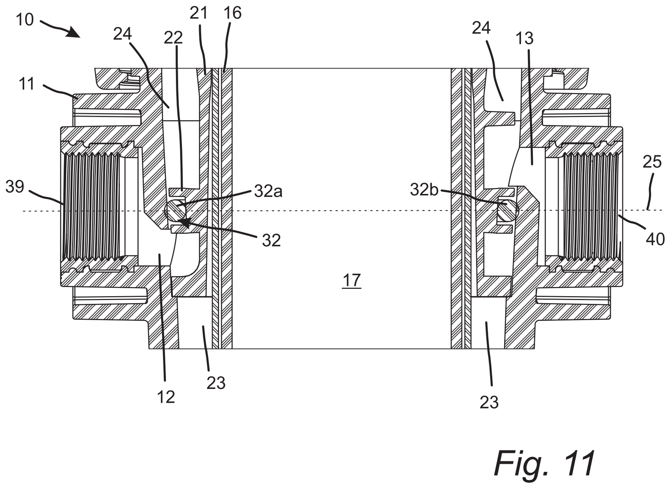

In Fig. 11, an alternative embodiment is shown. Fig. 11 is a partial section view of a UV-LED disinfection unit, taken in level with the inlet 39/outlet 40 connections of the unit 10. The differences of this embodiment compared to the earlier description, will be elaborated on below. Other components, parts and relationships therebetween are consistent with the earlier description.

The sleeve 21 comprises a separation means 22 which is horizontally arranged. The separation means 22 comprises a sealing 32. The first portion 32a of the sealing is arranged on the same vertical height as the second portion 32b of the sealing 32. When the sleeve 21 is arranged in the UV-LED disinfection unit 10, the first 32a and second 32b portions of the sealing 32 are located in-line with each other. In relation to a line 25, drawn through the centres of both the inlet connection 39 and the outlet connection 40, they may be located on the line 25.

The housing 11 comprises an inlet opening 12 for fluidly connecting the inlet connection 39 with the non-disinfected fluid chamber 23, and an outlet opening 13 for fluidly connecting the disinfected fluid chamber 24 with the outlet connection 40. The size (such as diameter or area) of the inlet 12 and outlet 13 openings, respectively, are smaller than the size (such as diameter or area) of the inlet 39 and outlet 40 connections, respectively.

The inlet opening 12 is provided in the vicinity of one end portion of the inlet connection 39. In relation to the line 25 drawn through the centres of both the inlet connection 39 and the outlet connection 40, the inlet opening 12 is arranged on a first side of this line 25. In Fig. 11, this corresponds to the inlet opening 12 being arranged below the line 25.

The outlet opening 13 is provided in the vicinity of an end portion of the outlet connection 40, being opposite to the portion of the inlet connection 39 in the vicinity of which the inlet opening 12 is provided. In relation to the line 25, the outlet opening 13 is arranged on a second side of the line 25. In Fig. 11, this corresponds to the outlet opening 13 being arranged above the line 25.

The separation of the non-disinfected fluid chamber 23 and the disinfected fluid chamber 24 is thus achieved, and an in-line installation of the UV-LED disinfection unit 10 is enabled.

The function of the UV-LED disinfection unit 10 will now be described with reference to Fig. 4.

The fluid to be disinfected enters the unit 10 via the inlet 39 and through the fluid inlet opening 12. The fluid enters the non-disinfected fluid chamber 23, where it is directed, by means of the separation means 22, towards the first portion 19 of the hollow structure 16. The fluid passes the flow distributor 29, which provides an advantageous flow distribution of the fluid into the fluid disinfection chamber 17.

The UV-LEDs 33 of the UV-LED unit 15 emits UV radiation, optionally UV-C radiation, into the fluid disinfection chamber 17, where the fluid is exposed to the radiation. The UV radiation disinfects the fluid as it travels through the fluid disinfection chamber 17 towards the second end portion 20 of the hollow structure 16.

Once the fluid reaches the second end portion 20, it flows via the fluid passage 38 between the UV-LED unit 15 and the inner wall of the reception means 42. The fluid thus contacts the exterior of the UV-LED unit 15. From the passage 38, the fluid enters the disinfected fluid chamber 24, where it is directed, by means of the separation means 22, towards the outlet opening 13, where it is discharged from the fluid disinfection unit 10.

When the fluid travels through the passage 38 around the UV-LED unit 15, it may contact all exterior surfaces of the UV-LED unit 15. This promotes cooling of the UV-LED unit 15. Especially, the portion of the UV-LED unit 15 housing the PCB 34 and the UV-LEDs 34 benefits from this, as the UV-LEDs produce heat. The fluid flowing past the UV-LED unit 15 promotes cooling of the UV-LED unit 15. The cooling paste provided between the UV-LED unit 15 casing 27 and the PCB 34 further promotes the cooling of the UV-LED unit 15.

The various embodiments described above are provided by way of illustration only and should not be construed to limit the invention. For example, the principles herein may be applied to any disinfection unit. Those skilled in the art will readily recognize various modifications and changes that may be made to the present invention without following the example embodiments and applications illustrated and described herein, and without departing from the scope of the present disclosure. For example, the fluid disinfection unit 10 may be mounted in any direction, such as horizontally, vertically, or anything in between. The fluid may enter the disinfection chamber 17 from the opposite way compared to what has been previously described herein.

The housing 11 has been described as being a two-part housing, comprising a container portion 35 and a cover portion 36. In other embodiments however, the housing may be a one-piece housing. The UV-LED unit 15 may be arranged in the opposite end portion of the hollow structure 16 compared to what has been previously described herein. The UV-LED unit 15 may be ring shaped, comprising a centre opening. In this embodiment, the fluid flows through said opening instead of around the UV-LED unit.

The fluid to be disinfected by means of the UV-LED fluid disinfection unit may be a liquid or a gas.

## Claims

1. A UV-LED fluid disinfection unit (10) comprising
- a housing (11) enclosing a cavity (14), and comprising an inlet connection (39) and an outlet connection (40), a fluid inlet opening (12) and a fluid outlet opening (13), wherein the fluid inlet opening (12) fluidly connects the inlet connection (39) and the cavity (14), and the fluid outlet opening (13) fluidly connects the cavity (14) and the outlet connection (40), and wherein the inlet connection (39) and the outlet connection (40) are arranged in-line with each other,
**characterized by** the UV-LED fluid disinfection unit (10) further comprising
- a hollow structure (16) comprising a lateral surface, a first end portion (19) and a second end portion (20), the hollow structure (16) being arranged in the cavity (14) such that said cavity (14) is divided into an inner chamber (17) and an outer chamber (18),
- a UV-LED unit (15) arranged in connection with one end portion (19, 20) of the hollow structure (16),
- a sleeve (21) arranged in the outer chamber (18), enclosing a portion of the lateral surface of the hollow structure (16), and/or the UV-LED unit (15), the sleeve (21) being provided with separation means (22) dividing the outer chamber (18) in a non-disinfected fluid chamber (23) and a disinfected fluid chamber (24) and comprising a sealing means (32), the sleeve (21) being arranged such that a first portion (32a) of the sealing means (32) is arranged adjacent a first side portion of the fluid inlet opening (12), and a second portion (32b) of the sealing means (32) is arranged adjacent a second side portion of the fluid outlet opening (13), being opposite said first side portion.

2. The UV-LED fluid disinfection unit (10) according to claim 1, wherein the sleeve (21) is arranged such that the first portion (32a) of the sealing means (32) is arranged above the fluid inlet opening (12), and the second portion (32b) of the sealing means (32) is arranged below the fluid outlet opening (13).

3. The UV-LED fluid disinfection unit (10) according to any one of the preceding claims, wherein the fluid inlet opening (12) is connected to the non-disinfected fluid chamber (23).

4. The UV-LED fluid disinfection unit (10) according to any one of the preceding claims, wherein the fluid outlet opening (13) is connected to the disinfected fluid chamber (24).

5. The UV-LED fluid disinfection unit (10) according to any one of the preceding claims, wherein the second portion (32b) of the sealing means (32) is circumferentially opposite the first portion (32a) of the sealing means (32).

6. The UV-LED fluid disinfection unit (10) according to any one of the preceding claims, wherein the UV-LED unit (15) comprises a casing (27) and a lid (28), wherein the lid (28) comprises at least one UV transparent portion.

7. The UV-LED fluid disinfection unit (10) according claim 6, wherein the casing (27) and the lid (28) form an enclosed space housing at least one or more of: one or more UV-LED(s) (33), and one or more PCB(s) (34).

8. The UV-LED fluid disinfection unit (10) according to claim 7, wherein the PCB (34) is provided with a cooling medium, optionally a cooling paste, optionally on a surface of the PCB (34) facing the casing (27).

9. The UV-LED fluid disinfection unit (10) according to any one of the preceding claims, wherein the UV-LED(s) (33) are configured to transmit UV light into the inner chamber (17), and wherein the inner chamber (17) is a fluid disinfection chamber.

10. The UV-LED fluid disinfection unit (10) according to any one of the preceding claims, wherein the separation means (22) comprises a flange (30) with a groove (31) housing the sealing means (32).

11. The UV-LED fluid disinfection unit (10) according to any one of the preceding claims, wherein the separation means (22) is provided obliquely around the sleeve (21).

12. The UV-LED fluid disinfection unit (10) according to any one of the preceding claims, wherein the sealing means (32) comprises on or more of: a ring-shaped sealing, optionally an O-ring or a sealing with any other suitable cross section geometry; an injection moulded sealing, a sealing integrally formed with the sleeve (21).

13. A method for disinfecting a fluid comprising:
- providing a UV-LED fluid disinfection unit (10) according to any one of the preceding claims,
- providing a flow of fluid through the inlet opening (12) into the non-disinfected fluid chamber (23),
- directing the fluid, by means of the separation means (22), towards one end portion (19) of the hollow structure (16), where the fluid enters the inner chamber (17), and flows toward the opposite end portion (20) of the hollow structure (16),
- disinfecting the fluid in the inner chamber (17) by means of UV radiation emitted from the UV-LED unit (15),
- directing the fluid into the disinfected fluid chamber (24),
- directing the fluid, by means of the separation means (22), towards the outlet opening (13).

14. The method for disinfecting a fluid according to claim 13, wherein the fluid contacts an exterior of the UV-LED unit (15), whereby the fluid cools the UV-LED unit (15).
